(19) Europäisches Patentamt / European Patent Office / Office européen des brevets

(11) **EP 3 205 329 B1**

(12) **EUROPEAN PATENT SPECIFICATION**

(45) Date of publication and mention
of the grant of the patent:
**11.12.2019 Bulletin 2019/50**

(21) Application number: **15849485.6**

(22) Date of filing: **07.10.2015**

(51) Int Cl.:
*A61K 8/25* (2006.01)    *A61K 8/06* (2006.01)
*A61K 8/73* (2006.01)    *A61Q 17/04* (2006.01)
*A61Q 19/00* (2006.01)    *A61K 8/27* (2006.01)
*A61K 8/29* (2006.01)

(86) International application number:
**PCT/JP2015/078469**

(87) International publication number:
**WO 2016/056589 (14.04.2016 Gazette 2016/15)**

(54) **OIL-IN-WATER-TYPE EMULSIFIED COSMETIC**

KOSMETIKUM MIT ÖL-IN-WASSER-EMULSION

PRODUIT COSMÉTIQUE ÉMULSIFIÉ DE TYPE HUILE DANS L'EAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.10.2014 JP 2014207326**

(43) Date of publication of application:
**16.08.2017 Bulletin 2017/33**

(73) Proprietor: **Sakai Chemical Industry Co., Ltd.**
**Sakai-shi, Osaka 590-8502 (JP)**

(72) Inventors:
• **ASHIDA, Takuro**
**Sakai-shi**
**Osaka 590-0985 (JP)**
• **MAGARA, Koichiro**
**Iwaki-shi**
**Fukushima 971-8183 (JP)**
• **SANO, Akifumi**
**Iwaki-shi**
**Fukushima 971-8183 (JP)**
• **SHIKE, Ayana**
**Iwaki-shi**
**Fukushima 971-8183 (JP)**

(74) Representative: **Isarpatent**
**Patent- und Rechtsanwälte Behnisch Barth Charles**
**Hassa Peckmann & Partner mbB**
**Friedrichstrasse 31**
**80801 München (DE)**

(56) References cited:
JP-A- H02 247 109        JP-A- H03 115 211
JP-A- H09 235 218        JP-A- H10 212 224
JP-A- 2000 319 128       JP-A- 2007 084 475
JP-A- 2009 161 496       JP-A- 2010 168 338
JP-A- 2011 073 971       JP-A- 2013 129 626

**Description**

TECHNICAL FIELD

The present invention relates to an oil-in-water emulsified cosmetic.

BACKGROUND OF THE DISCLOSURE

Recently, a suncare product has attracted attention because of raising public consciousness of ultraviolet. However, the inorganic UV filter is desired strongly because of safety, so suncut cosmetics containing inorganic powder microparticles such as titanium oxide, zinc oxide, and cerium oxide as a sunscreen agent are receiving a lot of attention. Further, in recent years, an O/W type suncut cosmetic is paid attention because the O/W cosmetic has a good touch feeling and is easy-to-remove. An aqueous dispersion of inorganic powder microparticles available to O/W type has been put on the market and particles primary treated with silica are used generally to block the surface activity of the inorganic powder microparticles and improve the hydrophilicity.

On the other hand, many of O/W type cosmetics which are commercially available contain a water-soluble polymer in an aqueous system and the polymer is carboxylic acid type or sulfonic acid type polymer which can impart a structural viscosity to water. These polymers have an anionic group such as carboxylic acid and sulfonic acid, and often impart a structural viscosity to the aqueous system by a high wettability of the above-mentioned group to maintain the stability of O/W type cosmetics.

When a water dispersion of inorganic powder microparticles primary treated with silica are contained in the O/W type cosmetics, the hydrophilicity of silanol group in silica is high so that the hydration part of the anionic group of the water-soluble polymer is affected to occur the viscosity decreasing and viscosity increasing of the aqueous system and gelling. Further, it causes a big trouble in the stability of O/W type cosmetics and decreases the touch feeling such as a strong creaking feeling due to silica.

Therefore, if a water dispersion of inorganic powder microparticles obtained by hydrophobic treating the outermost surface of silica-primary treated inorganic powder microparticles, the demerits mentioned above are quite improved but the influence of the silanol group can not be perfectly blocked. In Patent Document 1, a dispersion obtained by dispersing hydrophobic treated powders in water and an O/W emulsified cosmetic containing the same are disclosed. Further, the document discloses that a water-soluble polymer is used in combination. However, an attempt to improve the stability of emulsification system in which silica-primary treated inorganic powder microparticles are used has not been made. Further, the use of water-swelling substance is not specifically disclosed.

Patent Document 2 discloses inorganic powder microparticles having a primary coating layer containing silica with outermost layer subjected to a hydrophobic organic surface treatment. However, it is not specifically disclosed that the microparticles are used stably in a cosmetic together with the water-swelling substance.

It is desired to use a nonionic polymer having an effect to reduce the influence of silanol group of inorganic powder microparticles to the water-soluble polymer so as to achieve a high level of stability of O/W cosmetics. However, nonionic surfactans generally have a lower hydration ability than that of anionic surafactants so that it is hard to impart a high structural viscosity to the aqueous system.

Therefore, a water-swelling substance which is hardly affected by the silanol group of inorganic powder microparticles and can impart a high structural viscosity to the aqueous system has been desired.

The water-swelling substance may include water-swelling clay minerals such as vermiculite, and smectite, and water-swelling organic substances such as fine cellulose, and ager, and these substances are already used in cosmetics.

However, it is not known that the viscosity increasing or viscosity decreasing of the oil-in-water emulsified cosmetic can be suppressed and the stability can be improved by blending the water-swelling substances such as the water-swelling clay minerals, the fine cellulose and agar to oil-in-water emulsified cosmetics in which inorganic powder microparticles such as titanium oxide microparticles and zinc oxide microparticles are blended in the aqueous system. Especially in the oil-in-water emulsified cosmetic containing inorganic powder microparticles primary treated with a silica-containing treating agent with outermost surface subjected to a hydrophobic treatment in the aqueous system, it has never been known that the water-swelling substances have a great influence on the stability improvement, the touch feeling improvement, and the water repellency improvement.

PRIOR TECHNICAL DOCUMENTS

PATENT DOCUMENTS

[Patent Document 1] WO2013/018827
[Patent Document 2] WO2013/018828

JP 2009-161496 A describes a method for producing a cosmetic wherein the surface of metal oxide microparticles is coated with silica followed by making a hydrophobisation treatment on the outer side of the resulting surface.

JP 2011-073971 A discloses an oil-in-water type sunscreen cream that contains titanium oxide, which has been subjected to silica solution treatment and then to silicone solution treatment, and hectorite.

JP H03-115211 discloses a cosmetic for preventing ultraviolet light rays, having hydrophobic nature, containing powder prepared by coating titanium dioxide fine particles with a mixture comprising silicate hydrate and alumina hydrate, and wherein further the surface of the coated fine particles comprises silicone oil.

JP H02-247109 describes a cosmetic containing a powder prepared by subjecting titanium oxide of a generally spherical or irregular shape having a specific average particle diameter to coating treatment with a hydrated mixture consisting of hydrated silicic acid and hydrated alumina, and further coating the surface thereof with a silicone oil.

SUMMARY OF INVENTION

PROBLEM TO BE SOLVED BY THE INVENTION

The present disclosure relates to an oil-in-water emulsified cosmetic containing inorganic powder microparticles having a primary coating layer containing silica with outermost layer subjected to a hydrophobic treatment in the aqueous system, and the one object of the present disclosure is to provide an oil-in-water emulsified cosmetic with improved touch feeling, function, and stability by containing the specified water-swelling substances as aqueous thickener.

MEANS FOR SOLVING OBJECT

The present disclosure relates to an oil-in-water emulsified cosmetic containing inorganic powder micropaticles (A) having a primary coating layer containing silica with an outermost surface subjected to a hydrophobic organic surface treatment and at least one kind of water-swelling substance (B) wherein the coated amount of the primary coating layer is 1 to 25 % in terms of the whole inorganic materials relative to the whole inorganic powder,and wherein the water-swelling substance (B) is selected from the group consisting of hectorite, microorganism-produced cellulose, plant-derived cellulose, and agar. In the present disclosure, the water-swelling substance is nonionic and a substance which can increase the volume of itself by water intercalation or water absorbment

The water-swelling substance (B) is at least one selected from the group consisting of hectorite, microorganism-produced cellulose, plant-derived cellulose, and agar.

The inorganic powder microparticle is preferably at least one selected from the group consisting of titanium oxide, zinc oxide, and cerium oxide.

The hydrophobic organic surface treated inorganic powder microparticles (A) is preferably blended in the state of an aqueous dispersion obtained by disprsing the same in an aqueous system using an organopolysiloxane nonionic surfactant.

EFFECT OF THE INVENTION

The inventors found that, in an oil-in-water emulsified cosmetic containing inorganic powder microparticles having a primary coating layer containing silica with outermost surface subjected to a hydrophobic surface treatment in the aqueous system, as further outlined in present claim 1, a product which is superior in the long term stability and the touch feeling and has a high water repellency can be obtained without troubles such as viscosity decreasing, viscosity increasing, and gelling by blending one or more kinds of specified water-swelling substances as an aqueous thickener.

DETAILED DESCRIPTION OF PREFERRED EMBODIMENTS

The inorganic powder microparticles (A) to be used in the present disclosure have a primary coating layer containing silica. The primary coating layer may contain only silica, or may contain other inorganic materials. The other materials may include aluminium hydroxide, alumina, zirconia, and so on.

The primary coating layer containing silica may be formed by, for example, treating the inorganic powder surface with a treating agent containing Si source compound which can deposit $SiO_2$ on the powder surface after treating. Specifically, a wet method in which a Si source compound such as tetraalkoxysilane and sodium silicate is used and $SiO_2$ is deposited on the powder surface by hydrolysis or heat decomposition may be used. The Si source compound is preferably a compound which can be easily converted to $SiO_2$ such as tetraalkoxysilane and hydrolysis condensate thereof, sodium silicate, and potassium silicate. Two or more of them may be used in combination.

The coated amount of the primary coating layer is 1 to 25 wt%, more preferably 2 to 20 wt% in terms of the whole inorganic materials relative to the whole inorganic powder. When a compound which dissociates in water to generate polyvalent metal ion such as aluminium hydroxide is used, the used amount thereof is limited and preferably 10 wt% or less because an aluminium ion is eluted in aqueous system to decrease the viscosity of an anioninc water-soluble polymer.

The inorganic powder microparticle (A) is further subjected to a hydrophobic organic surface treatment on the outermost surface. A hydrophobic organic surface treating agent for treating the inorganic powder microparticle includes, for example, methylhydrogensiloxane polysiloxanes, dimethyl polysiloxanes, a copolymer of methylhydrogen polysiloxane and dimethyl polysiloxane, dimethyl polysiloxanes containing a reactive trialkoxysilyl group such as a trimethoxysilyl group and a triethoxysilyl group, alkyl-modified polysiloxanes containing the same reactive group, dimethyl polysiloxanes, alkylsilanes, alkyl titanates, and metal soaps. At least one compound of these compounds may be used in a known treatment method. By treating with the hydrophobic organic surface treating agent, the hydrophilic groups of the inorganic material for coating are blocked and the gel formation of the inorganic powder with the water-soluble polymer is suppressed so that the creaking feeling may be prevented at the time of use and water resistance may be improved.

In the hydrophobic organic treatment of the inorganic powder microparticle, from 2 to 15 wt% of the total amount of the inorganic powder after treatment is preferably subjected to the organic treatment. If the amount of the inorganic powder subjected to the organic treatment is less than 2 wt%, it is undesirable in view of insufficient hydrophobicity. If the amount is more than 15 wt%, it is undesirable because the effect of hydrophobicity is saturated. More preferably, from 4 to 12 wt% of the total amount of the inorganic powder is subjected to the organic treatment.

A primary particle diameter of inorganic powder microparticles (A) to be used in the present disclosure is preferably an average particle diameter of 5 to 200 nm which can generally show UV absorption/scattering effect. The lower limit is more preferably 10 nm, and the upper limit is more preferably 100 nm. In the specification, the particle diameter is measured by measuring the particle diameters of randomly selected 200 particles with an electron microscope, and calculating the average particle diameter of the primary particles.

The inorganic powder microparticle (A) is not particularly limited but may include titanium oxide, zinc oxide, and cerium oxide, and two or more kinds of them may be used in combination and composites thereof may be used.

As the shape of the inorganic powder microparticle (A), a powder having any shape can be used, including spherical, rod-shaped, needle-shaped, spindle-shaped, plate-shaped, and hexagonal plate-shaped particles, and needle-shaped aggregates. In rod-shaped, needle-shaped, or spindle-shaped particles, the average particle diameter is defined as the length in the direction of the short axis. In plate-shaped particles, the average particle diameter is defined as the average diameter of the maximum inscribed circle of the plane.

The oil-in-water emulsified cosmetic of the present disclosure preferably contains the inorganic powder microparticle (A) of 1 to 50 wt% in terms of inorganic powder, more preferably 3 to 20 wt%.

In the oil-in-water emulsified cosmetic of the present disclosure, the inorganic powder microparticle (A) is preferably present stably in aqueous phase. Thereby, the viscosity increasing/decreasing is not observed for a long term and a good stability can be achieved. Further, the inorganic powder microparticle (A) is free from aggregation so that a superior feeling in use, water repellency, an ultraviolet shielding effect, and visible light transparency can be imparted.

The oil-in-water emulsified cosmetic of the present disclosure contains at least one kind of water-swelling substance (B). The water-swelling substance (B) is nonionic and a substance which can increase the volume of itself by water intercalation or water absorbment. The water-swelling substance (B) is not a compound having thickening action derived from ionic function such as a carbomer and may develop thickening action by swelling when mixed with water. The water-swelling substance (B) according to the invention is selected from the group consisting of hectorite, microorganism-produced cellulose, plant-derived cellulose, and agar.

By compounding the water-swelling substance (B) in the oil-in-water emulsified cosmetic, a high structural viscosity can be imparted to the aqueous system and a stable oil-in-water emulsified cosmetic can be obtained without causing the viscosity decreasing and the viscosity increasing. Further, the obtained oil-in-water emulsified cosmetic has a good touch feeling without a sticky feeling and a creaking feeling and has a superior water repellency.

The water-swelling substance (B) is hardly affected by the silanol group of inorganic powder microparticles. The inorganic powder micropaticle (A) to be used in the oil-in-water emulsified cosmetic of the present disclosure has a primary coating layer containing silica and is subjected to a hydrophobic treatment on the outermost surface. Even if the outermost surface of the inorganic powder microparticle (A) is hydrophobic treated after the primary coating layer containing silica is formed, the silanol group in the silica may not be blocked perfectly. However, the excellent oil-in-water emulsified cosmetic as mentioned above can be obtained because the water-swelling substance (B) is hardly affected by the silanol group.

The water-swelling substance (B) is selected from the group consisting of hectorite, microorganism-produced cellulose, plant-derived cellulose (fine cellulose), and agar

The water-swelling clay mineral is hectorite. This compound is consisting of microparticles of micron or submicron orders, and generally have a layered structure. When mixed with water, water enters between the layers of a crystal so that the bottom interval expands. Finally, the particles of infinitely swollen state are dispersed in water.

The fine cellulose is fine celluloses having an average particle diameter of 5 μm or less, such as plant-derived cellulose being subdivided plant fiber and microorganism-produced cellulose which is produced by acetic acid bacteria and so on. The cellulose which is subdivided plant fiber is called nanofiber, and studied in various fields. There is a fine cellulose fiber obtained by oxidizing a hydroxyl group in the cellulose molecular and converting to an aldehyde group and a carboxyl group.

In addition, the microorganism-produced cellulose includes, for example, a cellulose fiber synthesized by fermenting a saccharide such as glycol by Acetobacter xylinum which is a type of acetic acid bacteria. This cellulose fiber is extremely fine fiber being 1/100 or less compared with conventional plant-drived celluloses. The fine cellulose fibers being 1 μm or less are entangled randomly with each other to form a three-dimensional network structure and the cellulose fibers having the three-dimensional network structure are dispersed in water.

The agar may include Ultra Agar AX-30 (manufactured by Ina Food Industry Co., Ltd), and Ultra Agar AX-100 (manufactured by Ina Food Industry Co., Ltd) as commercial products. As a commercial product containing agar and other polymers, Nomcort AG (manufactured by Nisshin Oillio Group, Ltd) is included.

Among these water-swelling substances, the fine cellulose is especially preferred because an addition effect can be significantly exerted. These celluloses have a three-dimensional network structure in which fine cellulose fibers are entangled randomly with each other so that a large structural viscosity can be imparted to the aqueous system. As the result, it is considered that the stability of the oil-in-water emulsified cosmetic containing the inorganic powder microparticles in aqueous system may be extremely improved. The fine cellulose is not affected by the silanol group of silica even if added in an oil-in-water emulsified cosmetic containing inorganic powder microparticles having a primary coating layer of silica in the aqueous system because the fine cellulose is nonionic polymer, so that the stability of the cosmetic can be maintained without the viscosity decreasing/increasing of the aqueous system. In addition, the fine cellulose is nonionic so that the obtained cosmetic does not cause the inferior touch feeling such as a sticky feeling and has a superior water resistance.

Further, because the water-swelling clay mineral causes charge deviation in a particle after swelling to increase the viscosity, so that it is subject to the addition of an acid, a base, and a salt because of the mechanism. However, the fine cellulose is chemically stable substance and has an excellent resistance to the addition of an acid and a basic substance. Therefore, the fine cellulose is preferred because the stability of the cosmetic containing it is easy to be maintained.

A suitable compounded amount of the water-swelling substance (B) is 0.01 to 15 wt% relative to the all amount of the cosmetic, and especially preferably 0.05 to 10 wt%.

The oil-in-water emulsified cosmetic of the present disclosure may contain a surfactant which is used conventionally in cosmetics.

An HLB value of the surfactant is preferably 8 to 9.5. When the HLB value is 8 to 9.5, a change with time of viscosity may be more suppressed, and a superior long term stability can be achieved. In the specification, the HLB value is calculated by the following expression as defined by W. C. Grifinn:

$$N_{HLB} = (E + P)/5$$

($N_{HLB}$: HLB value, E: wt% of a polyoxyethylene moiety based on the whole molecules of the dispersant, P: wt% of a polyhydric alcohol moiety based on the whole molecules of the dispersant) .

It is suitable that the total compounded amount of the surfactant is 0.01 to 5 wt% relative to the whole amount of the cosmetic when if two or more kinds of surfactants are used. The amount is more preferably 1 to 4 wt%. If the inorganic powder microparticles are compounded in the state of aqueous dispersion, and the nonionic surfactant contained in the aqueous dispersion corresponds to the surfactant having a low HLB as mentioned above (HLB is 9.5 or less), a compounded amount of the nonionic surfactant is contained in the total amount of surfactants having a low HLB (HLB is 9.5 or less).

The oil-in-water emulsified cosmetic of the present disclosure preferably contains a surfactant having a high HLB (HLB is 10.0 or more). By containing such surfactants, the emulsified state can be kept stably. The surfactant with a high HLB may include an anionic surfactant, a cationinc surfactant, a nonionic surfactant, and an ampholytic surfactant, and is not limited. Any surfactants may be used if the surfactant is used in conventional cosmetics. A compounded amount is preferably 0.1 to 20 wt% relative to the whole amount of the cosmetic, especially preferably 0.2 to 10 wt%.

The nonionic surfactant may include a polyglycerin fatty acid ester with a high HLB (HLB is 10.0 or more), a polyoxyalkylene glycerin fatty acid ester with a high HLB, a polyoxyalkylene sorbitan fatty acid ester, a polyoxyalkylene sorbitol fatty acid ester, a polyoxyalkylene fatty acid ester, a polyoxyalkylene hydrogenated castor oil, a polyoxyalkylene sterol, a polyoxyalkylene alkyl ether, and polyoxyalkylene alkyl ether phosphoric acid,

Further, the oil-in-water emulsified cosmetic of the present disclosure preferably contains an organopolysiloxane nonionic surfactant. The inorganic powder microparticles (A) can be dispersed more suitably in aqueous phase by containing the organopolysiloxane nonionic surfactant. The organopolysiloxane nonionic surfactant is not particularly limited but includes the compounds as mentioned below. A compounded amount of the organopolysiloxane nonionic surfactant is not particularly limited but is preferably 0.1 to 10 wt%. The organopolysiloxane nonionic surfactant may be a surfactant with a high HLB (HLB is 10.0 or more).

The oil-in-water emulsified cosmetic of the present disclosure contains an oil phase. An oil contained in the oil phase is not particularly limited but includes avocado oil, camellia oil, turtle oil, macadamia nut oil, corn oil, mink oil, olive oil, rapeseed oil, egg-yolk oil, sesame oil, persic oil, wheat germ oil, sasanqua oil, castor oil, linseed oil, safflower oil, cotton seed oil, perilla oil, soybean oil, arachis oil, tea seed oil, kaya oil, rice bran oil, Japanese tung oil, jojoba oil, germ oil, triglycerol, glyceryl trioctanoate, glyceryl triisopalmitate, cacao butter, coconut oil, horse fat, palm oil, beef tallow, mutton tallow, hydrogenated beef tallow, palm kernel oil, lard, beef bone fat, hydrogenated oil, neatsfoot oil, Japan wax, hydrogenated castor oil, beeswax, candelilla wax, cotton wax, carnauba wax, bayberry wax, insect wax, spermaceti wax, montan wax, bran wax, lanolin, kapok wax, lanolin acetate, liquid lanolin, sugarcane wax, isopropyl lanolin fatty acid, hexyl laurate, reduced lanolin, hard lanolin, shellac wax, POE lanolin alcohol ether, POE lanolin alcohol acetate, POE cholesterol ether, polyethylene glycol lanolin fatty acid, POE hydrogenated lanolin alcohol ether, liquid paraffin, ozokerite, pristane, paraffin, ceresin, squalene, Vaseline, and microcrystalline wax.

Aqueous components and oily components to be generally used for cosmetics other than the above-mentioned components, surfactants other than the above-metioned surfactants, polyhydric alcohols, powders, ultraviolet absorbers, preservatives, antibacterial agents, antioxidants, beauty components, and perfumes may be added.

The oil-in-water cosmetic of the present disclosure is not particularly limited, but includes skin care products, hair care products, and make up products. Among them, the cosmetic may be used suitably as a foundation, a sunscreen agent, and a makeup base. A form of the cosmetic is not particularly limited but may be a liquid form, an emulsion form, a cream form, a solid form, a paste form, a gel form, a multilayer form, a mousse form, a spray form, and so on.

A method for producing the oil-in-water emulsified cosmetic of the present disclosure is not particularly limited but a known emulsification method may be used. For example, a method comprising stirring an aqueous phase and an oily phase uniformly respectively, heating them, and adding the oil phase to the aqueous phase to emulsify may be used.

The method for adding the inorganic powder microparticle (A) to the aqueous phase is not particularly limited but a method comprising preparing an aqueous dispersion of the inorganic powder microparticle (A) dispersed in water, and mixing with another components to prepare an oil-in-water emulsified cosmetic by a conventional method is preferred. By this method, the oil-in-water emulsified cosmetic of the present disclosure can be obtained stably.

The aqueous dispersion preferably contains the inorganic powder microparticle (A), a polyhydric alcohol, water and a nonionic surfactant.

The aqueous dispersion preferably contains the inorganic powder of 30 wt% or more relative to the total amount of the dispersion. The amount is preferably within the above-mentioned range because a small amount of the dispersion can express an effect as a cosmetic material. The amount is more preferably 40 wt% or more. The upper limit of the amount is not particularly limited but is preferably 70 wt% or less, more preferably 60 wt% or less.

The polyhydric alcohol may be one or more polyhydric alcohol selected from the group consisting of propylene glycol, butylene glycol, pentane diol, dipropylene glycol, hexanediol, and heptanediol, and butylene glycol, pentane diol, dipropylene glycol, and hexanediol are especially preferred.

An amount of the polyhydric alcohol is 5 to 25 wt% in the aqueous dispersion. The lower limit thereof is more preferably 8 wt%, and the upper limit thereof is more preferably 20 wt%. The nonionic surfactant can be uniformly orientated on the powder surface to stabilize the dispersion by blending the polyhydric alcohol.

The nonionic surfactant may be used singly or two or more of them may be used in combination. When the nonionic surfactant is mixed with 1,3-butylene glycol with a concentration of 20 wt%, it is preferred that the nonionic surfactant is dissolved transparently or slightly muddy at 35°C (when the nonionic surfactant is paste form or solid at room temperature, the condition is confirmed at 35°C after heating to make uniform).

The "dissolved transparently" means that a haze value of the obtained mixture at an optical path length of 10 mm and 35°C is less than 10. "Dissolved slightly muddy" means that a haze value of the obtained mixture at an optical path length of 10 mm and 35°C is 10 or more, and the total light transmittance is 30% or more.

By using the nonionic surfactant, a layer of the nonionic surfactant can be formed on the particle surface efficiently. An amount of the nonionic surfactant is preferably 1 to 25 wt% relative to the total amount of the dispersion. The lower limit is more preferably 2 wt%, and still more preferably 3 wt%. The upper limit is more preferably 20 wt%, more preferably 15 wt%.

When the nonionic surfactant is mixed with water with a concentration of 20 wt%, the surfactant is preferably insoluble or dispersed cloudy (when the nonionic surfactant is paste form or solid at room temperature, the condition is confirmed at 35°C after heating to make uniform). In the case of using two or more kinds of nonionic surfactants in combination, a mixture of two or more nonionic surfactants obtained by mixing them at the same ratio as the mixing ratio in the water-based dispersion is necessarily insoluble or dispersed cloudy when the above-mentioned test is carried out.

In the specification, "insoluble" means that an unmelted residue is caused in the mixture, or a phase separation is occurred an hour later even if the mixture looks muddy. "Dispersed cloudy" means that a haze value of the obtained mixture at an optical path length of 10 mm and 35°C is 10 or more, and the total light transmittance is less than 30%.

An HLB value of the surfactant is preferably 6 or more and 12 or less. When a dispersion which is obtained by dispersing with the use of only the nonionic surfactant having an HLB value of over 12 is blended in an O/W product, the solubility of the nonionic surfactant to water is too high so that the adsorption ability to the powder becomes ungood. Further, the viscosity increasing of the product tends to occur and the water resistance tends to deteriorate. When the HLB value of the nonionic surfactant to be used is less than 6, a water dispersion cannot be prepared or the stability is bad even if a water dispersion can be obtained.

The nonionic surfactant may include a polyoxyalkylene alkyl ether, a polyoxyalkylene fatty acid ester, a polyoxyalkylene sorbitan fatty acid ester, a polyoxyalkylene castor oil, a polyoxyalkylene hydrogenated castor oil, a polyoxyalkylene sorbitol tetra fatty acid ester, a glycerin fatty acid ester, a sorbitan fatty acid ester, a polyglycerin fatty acid ester, a sucrose fatty acid esters, an organopolysiloxane having a polyoxyalkylene group, an organopolysiloxane having a polyglycerin group, and an organopolysiloxane having a sugar chain.

Among them, a polyoxyalkylene fatty acid ester, a polyoxyalkylene sorbitan fatty acid ester, and an organopolysiloxane having a polyoxyalkylene group are preferably used as the nonionic surfactant.

Further, the organopolysiloxane nonionic surfactant as mentioned above is most preferred. When the organopolysiloxane nonionic surfactant is used, the especially excellent effects such as a good stability of a composition and a good feeling in use as a cosmetic can be given.

A dispersing method for preparing the aqueous dispersion may include known methods such as a method using a bead mill, a jet mill or a high-pressure homogenizer which can disperse uniformly.

The aqueous dispersion may contain an antiseptic preservative and an antibacterial agent such as alkyl ester of paraoxybenzoic acid, benzoic acid, sodium benzoate, sorbic acid, potassium sorbate, phenoxyethanol, salicylic acid, lime acid, sorbic acid, p-chlorometacresol, hexachlorophen, benzalkonium chloride, chlorhexidine chloride, photosentive element, and phenoxyethanol, unless the performance of the dispersion is deteriorated.

A method for producing the oil-in-water emulsified cosmetic of the present disclosure using the aqueous dispersion is not particularly limited but may include a general emulsification operation using a homogenizer after mixing raw materials.

EXAMPLES

The present invention will now be described in more detail with reference to the examples. Unless otherwise noted, in the examples and comparative examples, "%" means wt%.

[Water dispersion I: Production of water dispersion of titanium oxide]

KF-6013 (PEG-9 dimethicone: manufactured by Shin-Etsu Chemical Co., Ltd.: HLB 10.0) 10.0 g and silicone surface-treated, silica surface-treated titanium oxide microparticles (STR-100W-LPT manufactured by Sakai Chemical Industry Co., Ltd.: spindle-shaped particles having a short axis of 20 nm and a long axis of 100 nm) 40.0g were mixed with 1,3-butylene glycol 10.0 g. Then, water 40.0g was added and stirred to obtain a water dispersion of titanium oxide I.

[Water dispersion II: Production of water dispersion of zinc oxide]

KF-6013 (PEG-9 dimethicone: manufactured by Shin-Etsu Chemical Co., Ltd. : HLB 10.0) 5.0gand silicone surface-treated, silica surface-treated zinc oxide microparticles (FINEX-33W-LP2 manufactured by Sakai Chemical Industry Co., Ltd. : average particle diameter of 35 nm) 60.0g were mixed with 1,3-butylene glycol 15.0g. Then, water 20.0g was added and stirred to obtain a water dispersion of zinc oxide II.

(Examples 1 to 7, and comparative examples 1 to 6)

Sunscreen O/W creams of examples 1 to 7, and comparative examples 1 to 6 having the compositions as shown in tables 1 and 2 were prepared by using the prepared water dispersion of titanium oxide I, and the qualities thereof were evaluated.

(Production method)

A: The components 1-4 are mixed and heated.
B: The components 5-17 are mixed uniformly and heated.
C: A is added to B to emulsify and then cooled to obtain each sunscreen O/W cream (examples 1 to 7 and comparative examples 1 to 6).

The evaluations were done according to the following method.

1. Stability of sunscreen O/W cream

After the prepared O/W cream was left to stand at 50°C for a month and three months, it was confirmed whether or not the viscosity of the cream was increased or decreased, respectively.

[Evaluation criteria]

◎: The viscosity is not increased or decreased.
○: The viscosity is slightly increased or decreased.
Δ: The viscosity is increased or decreased.
✕: The viscosity is remarkably increased to cause gelling, or remarkably decreased so that the cream was converted to a liquid state.

2. Evaluation of feeling in use

After a use test was done by 20 specialized panels for evaluation, the spreading and elongation at coating, the sticky feeling and the creaking feeling of skin after coating, the durability to sweat and water, and the sunscreen effect were evaluated on a scale of one to five based on the following criteria and then the average scores were determined to decide.

5 points: excellent good
4 points: good
3 points: normal
2 points: slightly ungood
1 point: ungood

[Decision]

◎: average score of 4.5 or more
○: average score of 3.5 or more and less than 4.5
Δ: average score of 2.5 or more and less than 3.5
✕: average score of less than 2.5

3. Total evaluation

◎: Stability and feeling in use are extremely excellent.
○: The viscosity is slightly increased or slightly decreased, or feeling in use is slightly inferior.
Δ: The viscosity is increased or decreased, or feeling in use is inferior.
✕: Stability is ungood and feeling in use is inferior.

Table 1

|  |  | Ex. 1 | Ex. 2 | Ex. 3 | Ex. 4 | Ex. 5 | Ex. 6 | Ex. 7 |
|---|---|---|---|---|---|---|---|---|
| 1 | Hydrogenated polyisobutene | 20.7 | 20.7 | 20.7 | 20.7 | 20.7 | 20.7 | 20.7 |
| 2 | Behenyl alcohol | 1.0 | 1.0 | 1.0 | 1.0 | 1.0 | 1.0 | 1.0 |
| 3 | Sorbitan sesquioleate (HLB 3.7) | 0.5 | 0.5 | 0.5 | 0.5 | 0.5 | 0.5 | 0.5 |

(continued)

|  |  | Ex. 1 | Ex. 2 | Ex. 3 | Ex. 4 | Ex. 5 | Ex. 6 | Ex. 7 |
|---|---|---|---|---|---|---|---|---|
| 4 | Glyceryl stearate (SE) (HLB 11.6) | 0.8 | 0.8 | 0.8 | 0.8 | 0.8 | 0.8 | 0.8 |
| 5 | (Acrylate/alkyl acrylate (C10-C30))crosspolymer | 0.1 | 0.1 | 0.1 | 0.05 | 0.05 | 0.05 | 0.05 |
| 6 | Carbomer | - | - | - | 0.05 | 0.05 | 0.05 | 0.05 |
| 7 | Polysorbate 80 (HLB 15.0) | 1.5 | 1.5 | 1.5 | 1.5 | 1.5 | 1.5 | 1.5 |
| 8 | Hectorite | 0.5 | - | - | 0.5 | - | - | 0.2 |
| 9 | Microorganism-produced cellulose | - | 0.5 | - | - | 0.5 | - | 0.3 |
| 10 | Agar | - | - | 0.2 | - | - | 0.2 | - |
| 11 | Xanthan gum | - | - | - | - | - | - | - |
| 12 | Hydroxyethyl cellulose | - | - | - | - | - | - | - |
| 13 | Carboxymethyl cellulose | - | - | - | - | - | - | - |
| 14 | TEA | 0.1 | 0.1 | 0.1 | 0.1 | 0.1 | 0.1 | 0.1 |
| 15 | 1,3-BG | 8.0 | 8.0 | 8.0 | 8.0 | 8.0 | 8.0 | 8.0 |
| 16 | Water dispersion of titanium oxide I | 25.0 | 25.0 | 25.0 | 25.0 | 25.0 | 25.0 | 25.0 |
| 17 | Purified water | 41.8 | 41.8 | 42.1 | 41.8 | 41.8 | 42.1 | 41.8 |
|  | Stability after a month | ◎ | ◎ | ◎ | ◎ | ◎ | ◎ | ◎ |
|  | Stability after three months | ○ | ◎ | ○ | ○ | ◎ | ○ | ◎ |
|  | Feeling in use | ◎ | ◎ | ◎ | ◎ | ◎ | ◎ | ◎ |
|  | Total evaluation | ○ | ◎ | ○ | ○ | ◎ | ○ | ◎ |

Table 2

|  |  | Compar. ex. 1 | Compar. ex. 2 | Compar. ex. 3 | Compar. ex. 4 | Compar. ex. 5 | Compar. ex. 6 |
|---|---|---|---|---|---|---|---|
| 1 | Hydrogenated polyisobutene | 20.7 | 20.7 | 20.7 | 20.7 | 20.7 | 20.7 |
| 2 | Behenyl alcohol | 1.0 | 1.0 | 1.0 | 1.0 | 1.0 | 1.0 |
| 3 | Sorbitan sesquioleate (HLB 3.7) | 0.5 | 0.5 | 0.5 | 0.5 | 0.5 | 0.5 |
| 4 | Glyceryl stearate (SE) (HLB 11.6) | 0.8 | 0.8 | 0.8 | 0.8 | 0.8 | 0.8 |
| 5 | (Acrylate/alkylacrylate (C10-C30)) crosspolymer | 0.2 | 0.05 | 0.05 | 0.05 | 0.05 | 0.05 |
| 6 | Carbomer | - | 0.05 | 0.05 | 0.05 | 0.05 | 0.05 |
| 7 | Polysorbate 80 (HLB 15.0) | 1.5 | 1.5 | 1.5 | 1.5 | 1.5 | 1.5 |
| 8 | Hectorite | - | - | - | - | - | - |
| 9 | Microorganism-produced cellulose | - | - | - | - | - | - |
| 10 | Agar | - | - | - | - | - | - |
| 11 | Xanthan gum | - | - | 0.1 | - | - | 0.05 |

(continued)

| | | | Compar. ex. 1 | Compar. ex. 2 | Compar. ex. 3 | Compar. ex. 4 | Compar. ex. 5 | Compar. ex. 6 |
|---|---|---|---|---|---|---|---|---|
| 12 | Hydroxyethyl cellulose | | - | - | - | 0.1 | - | 0.05 |
| 13 | Carboxymethyl cellulose | | - | - | - | - | 0.1 | - |
| 14 | TEA | | 0.2 | 0.1 | 0.1 | 0.1 | 0.1 | 0.1 |
| 15 | 1,3-BG | | 8.0 | 8.0 | 8.0 | 8.0 | 8.0 | 8.0 |
| 16 | Water dispersion of titanium oxide I | | 25.0 | 25.0 | 25.0 | 25.0 | 25.0 | 25.0 |
| 17 | Purified water | | 42.1 | 42.3 | 42.2 | 42.2 | 42.2 | 42.2 |
| | Stability after a month | | ○ | ○ | ○ | ○ | ○ | ○ |
| | Stability after three months | | × | × | × | Δ | × | Δ |
| | Feeling in use | | ○ | ○ | ◎ | ◎ | ◎ | ◎ |
| | Total evaluation | | × | × | × | Δ | × | Δ |

As is evident from the result of table 1, as for the suncsreen O/W creams of the examples 1 to 7 and comparative examples 1 to 6 in which the prepared water dispersion of titanium oxide I was used, the O/W creams of examples 1 to 7 are stable without the viscosity increasing and decreasing after one month at 50°C. Further, the creams are extremely stable since the viscosity change is not occurred, or the viscosity is slightly increased or slightly decreased even after three months. Furthermore, as for the feeling in use thereof, the elongation and spreading at coating are excellent and creaking feeling of skin after coating is not occurred, and the obtained cream is found to be a O/W cream having a superior water resistance and an extremely high sunscreen effect. The O/W creams of examples 2, 5, and 7 obtained by using the microorganism-produced cellulose are found to be creams having an extremely high stability without the viscosity change even after three months at 50°C.

As for comparative examples 1 to 6, there are some opinions that the sticky feeling and the creaking feeling are occurred more than examples and some creams were evaluated as score of 3 or 2 in the sections of "the sticky feeling of the skin after coating" and "the creaking feeling". However, in the sections of "the spreading and elongation at coating", "durability to sweat and water" and "the sunscreen effect", the scores of creams of comparative examples were equal to that of examples. However, as for the stability, the viscosity of the creams of comparative examples 1 to 6 are slightly increased or slightly decreased after one month at 50°C. Further, after three months, the viscosity of creams of comparative examples 1, 2, 3, and 5 in which only anionic polymer is used is increased remarkably to become a gel, and the viscosity of the creams of comparative examples 4 and 6, in which nonionic polymers are compounded, is decreased significantly and the reason may be the low structural viscosity of the aqueous system.

Taking every factor into consideration, the O/W creams of examples 1 to 7 containing the water-swelling substances such as a water-swelling clay mineral, a microorganism-produced cellulose, and agar are superior in the stability and the feeling in use. Especially, the O/W creams of examples 2, 5, and 7 containing the microorganism-produced cellulose have especially stability and the viscosity increasing or decreasing is not occurred after three months at 50°C. On the other hand, the creams of comparative examples 1 to 6 containing a conventional anionic polymer or nonionic polymer are not too inferior in the feeling in use compared with creams of examples, but the stability with time are clearly inferior because the viscosity increasing or decreasing is shown clearly and some creams become gel or liquid.

O/W cream products of the following compositions were prepared.

[Example 8] (O/W cream)

| | (Components) | (%) |
|---|---|---|
| 1 | Isotridecyl isononanoate | 15.8 |
| 2 | Three-dimentional crosslinked silicone (※1) | 4.0 |

(continued)

| | (Components) | (%) |
|---|---|---|
| 3 | Polyglyceryl isostearate-4 (HLB: 8.2) | 0.2 |
| 4 | Acrylate/alkyl acrylate (C10-C30) crosspolymer | 0.1 |
| 5 | Plant-derived cellulose (2% aq) (※2) | 5.0 |
| 6 | Hydroxyethyl cellulose | 0.2 |
| 7 | Triethanolamine | 0.1 |
| 8 | 1, 3-butylene glycol | 5.0 |
| 9 | Water dispersion of titanium oxide I | 5.0 |
| 10 | Purified water | 64.6 |

(※1)Three-dimentional crosslinked silicone: 9040 Silicone Elastomer Blend (manufactured by Dow Corning TORAY)

(※2)Plant-derived cellulose: RHEOCRYSTA C-2SP (manufactured by DKS Co. Ltd.)

(Production method)

A: The components 1-3 are mixed uniformly.
B: The components 4-10 are mixed uniformly.
C: A is added to B and the resultant is emulsified to obtain an O/W cream.

The O/W cream of the example 8 obtained as mentioned above is stable without a change over time, and the cream can spread smoothly on skin at coating and does not cause a sticky feeling and creaking feeling after coating. So, it is an O/W cream having a superior dry feeling.

[Example 9] (Sunscreen O/W cream)

| | (Components) | (%) |
|---|---|---|
| 1 | Isotridecyl isononanoate | 15.8 |
| 2 | Ethylhexyl methoxycinnamate | 5.0 |
| 3 | Hexyl diethylaminohydroxybenzoylbenzoate | 2.0 |
| 4 | Glyceryl tri(behenate/eicosadioate) (※3) | 0.5 |
| 5 | Behenyl alcohol | 1.0 |
| 6 | Sorbitan sesquiisostearate (HLB 4.5) | 0.5 |
| 7 | Glyceryl stearate (SE) | 0.7 |
| 8 | Polyglyceryl distearate-10 (HLB 9.5) | 0.5 |
| 9 | Carbomer | 0.1 |
| 10 | Acrylate/alkyl acrylate (C10-C30) crosspolymer | 0.1 |
| 11 | Agar (※4) | 0.2 |
| 12 | PEG-20 sorbitan isostearate (HLB 15.0) | 1.5 |
| 13 | Triethanolamine | 0.2 |
| 14 | 1, 3-butylene glycol | 5.0 |
| 15 | Ethanol | 5.0 |
| 16 | Water dispersion of titanium oxide I | 25.0 |

(continued)

| | (Components) | (%) |
|---|---|---|
| 17 | Purified water | 36.9 |

(※3)Glyceryl tri(behenate/eicosadioate) : NOMCORT HK-G (manufactured by Nisshin Oillio Group, Ltd)

(※4) Agar : Ultra agar AX-100 (manufactured by Ina Food Industry Co., Ltd)

(Production method)

A: The components 1-8 are mixed uniformly and heat dissolved.
B: The components 9-17 are mixed uniformly and heated.
C: A is added to B and the resultant is emulsified. The emulsion was cooled to obtain a sunscreen O/W cream.

The sunscreen O/W cream of the example 9 obtained as mentioned above is stable without change over time, and can spread smoothly on skin at coating, and does not cause a sticky feeling and creaking feeling after coating. Further, the sunscreen O/W cream has a high transparency, suppresses whiteness, and is superior in maintaining the sunscreen effect.

[Example 10] (Sunscreen O/W cream)

| | (Components) | (%) |
|---|---|---|
| 1 | Hydrogenated polyisobutene | 14.5 |
| 2 | Ethylhexyl methoxycinnamate | 5.0 |
| 3 | Zinc oxide microparticle//propylene glycol dicaprate dispersion (※5) | 15.0 |
| 4 | Polyglyceryl diisostearate-6 (HLB: 8) | 0.5 |
| 5 | (Hydroxyethyl acrylate/sodium acryloyldimethyl taurate) copolymer (※6) | 0.8 |
| 6 | PEG-20 sorbitan isostearate | 1.0 |
| 7 | Plant-derived cellulose (2% aq) (※2) | 4.0 |
| 8 | 1, 3-butylene glycol | 8.0 |
| 9 | Water dispersion of titanium oxide I | 25.0 |
| 10 | Purified water | 26.2 |

(※5)Zinc oxide microparticle/propylene glycol dicaprate dispersion : FINEX 30S-LPT (manufactured by Sakai Chemical Industry Co., Ltd., : average particle diameter 35 nm) 65 g, polyhydroxystearic acid 2 g, and propylene glycol dicaprylate 33 g were mixed and dispersed.

(※6) (Hydroxyethyl acrylate/sodium acryloyldimethyl taurate) copolymer : SEPINOV EMT 10 (manufactured by SEPPIC)

(Production method)

A: The components 1-4 are mixed.
B: The components 5-10 are mixed uniformly.
C: A is added to B to obtain a sunscreen O/W cream.

The sunscreen O/W cream of the example 10 obtained as mentioned above is stable without change over time, and can spread smoothly on skin at coating, and does not cause a sticky feeling and creaking feeling after coating. Further, the sunscreen O/W cream has a high transparency, suppresses whiteness, and is superior in maintaining the sunscreen effect.

[Example 11] (Sunscreen O/W cream)

| | (Components) | (%) |
|---|---|---|
| 1 | Isotridecyl isononanoate | 14.8 |
| 2 | Ethylhexyl methoxycinnamate | 5.0 |
| 3 | Hexyl diethylaminohydroxybenzoylbenzoate | 2.0 |
| 4 | Three-dimentional crosslinked silicone (※1) | 4.0 |
| 5 | Behenyl alcohol | 1.0 |
| 6 | Sorbitan sesquiisostearate (HLB 4.5) | 0.5 |
| 7 | Glyceryl stearate (SE) | 0.7 |
| 8 | PEG-20 glyceryl tristearate (HLB: 8) | 0.5 |
| 9 | (Sodium acrylate/sodium acryloyldimethyl taurate) copolymer (※7) | 1.0 |
| 10 | Plant-derived cellulose (2% aq) (※2) | 6.0 |
| 11 | Hydroxyethyl cellulose (2% aq) | 10.0 |
| 12 | PEG-20 sorbitan isostearate | 1.5 |
| 13 | PEG-12 isostearate (HLB 12) | 3.0 |
| 14 | 1, 3-butylene glycol | 10.0 |
| 15 | Titanium oxide microparticle (※8) | 10.0 |
| 16 | Purified water | 30.0 |

(※7) (Sodium acrylate/sodium acryloyldimethyl taurate) copolymer: SIMULGEL EG (manufactured by SEPPIC)

(※8)Titanium oxide microparticle: STR-100W-LPT (silicone surface-treated, silica surface-treated titanium oxide microfine particles: spindle-shaped particles having a particle diameter of 20 nm in the direction of the short axis and 100 nm in the direction of the long axis) (manufactured by Sakai Chemical Industry Co., Ltd.,)

(Production method)

A: The components 1-8 are mixed and heated.
B: After the components 12-15 are mixed uniformly, the components 9-11 and 16 are added and heated.
C: A is added to B and the resultant is emulsified. The emulsion was cooled to obtain a sunscreen O/W cream.

The sunscreen O/W cream of the example 11 obtained as mentioned above is stable without change over time, and can spread smoothly on skin at coating, and does not cause a sticky feeling and creaking feeling after coating. Further, the sunscreen O/W cream has a high transparency, suppresses whiteness, and is superior in maintaining the sunscreen effect.

[Example 12] (Sunscreen O/W cream)

| (Components) | | (%) |
|---|---|---|
| 1 | Hydrogenated polyisobutene | 18.6 |
| 2 | Three-dimentional crosslinked silicone (※1) | 4.5 |
| 3 | Behenyl alcohol | 0.7 |
| 4 | Sorbitan sesquiisostearate | 0.5 |
| 5 | Glyceryl stearate (SE) | 0.7 |
| 6 | Polyglyceryl isostearate-4 (HLB: 8.2) | 0.5 |
| 7 | (Sodium acrylate/sodium acryloyldimethyl taurate) copolymer (※7) | 1.2 |
| 8 | Plant-derived cellulose (2% aq) (※2) | 5.0 |
| 9 | PEG-20 sorbitan isostearate | 1.5 |
| 10 | 1, 3-butylene glycol | 5.0 |
| 11 | Ethanol | 5.0 |
| 12 | Water dispersion of titanium oxide I | 20.0 |
| 13 | Water dispersion of zinc oxide II | 25.0 |
| 14 | Purified water | 11.8 |

(Production method)

A: The components 1-6 are mixed and heated.
B: The components 7-14 are mixed uniformly and heated.
C: A is added to B and the resultant is emulsified. The emulsion

was cooled to obtain a sunscreen O/W cream. The sunscreen O/W cream of the example 11 obtained as mentioned above is stable without change over time, and can spread smoothly on skin at coating, and does not cause a sticky feeling and creaking feeling after coating even though the cream has a system in which zinc oxide and titanium oxide are mixed and dispersed. Further, the obtained cream is an organic ultraviolet absorbent free sunscreen O/W cream having a high transparency, an effect of suppressing whiteness, and a superior water resistance, and being superior in maintaining the sunscreen effect.

INDUSTRIAL APPLICABILITY

The present disclosure can provide an oil-in-water emulsified cosmetic being superior in long term stability and having a good touch feeling and a high resistance to water and sweat, and which can impart an ultraviolet blocking effect.

**Claims**

1. An oil-in-water emulsified cosmetic containing
   inorganic powder micropaticles (A) having a primary coating layer containing silica with an outermost surface subjected to a hydrophobic organic surface treatment and
   at least one kind of water-swelling substance (B),
   wherein the coated amount of the primary coating layer is 1 to 25 % in terms of the whole inorganic materials relative to the whole inorganic powder,and
   wherein the water-swelling substance (B) is selected from the group consisting of hectorite, microorganism-produced cellulose, plant-derived cellulose, and agar.

2. The oil-in-water emulsified cosmetic according to claim 1,
   wherein the inorganic powder microparticle is at least one selected from the group consisting of titanium oxide, zinc oxide, and cerium oxide.

3. The oil-in-water emulsified cosmetic according to any one of claims 1 or 2,

wherein the hydrophobic organic surface treated inorganic powder microparticles (A) is blended in the state of a aqueous dispersion obtained by disprsing the same in an aqueous system using an organopolysiloxane nonionic surfactant.

**Patentansprüche**

1. Öl-in-Wasser-emulgiertes Kosmetikum, enthaltend
anorganische Pulvermikroteilchen (A), die eine primäre Beschichtungsschicht aufweist, die Silizium enthält, mit einer äußersten Oberfläche, die einer hydrophoben organischen Oberflächenbehandlung unterzogen wird, und mindestens eine Art von wasserquellbarer Substanz (B),
wobei der beschichtete Betrag der primären Beschichtungsschicht 1 bis 25 % im Hinblick auf die gesamten anorganischen Materialien relativ zu dem gesamten anorganischen Pulver beträgt, und
wobei die wasserquellbare Substanz (B) aus der Gruppe ausgewählt ist bestehend aus Hectorit, Mikroorganismuserzeugter Cellulose, pflanzlicher Cellulose und Agar.

2. Öl-in-Wasser-emulgiertes Kosmetikum nach Anspruch 1,
wobei das anorganische Pulvermikroteilchen mindestens eines ist, das aus der Gruppe ausgewählt ist bestehend aus Titanoxid, Zinkoxid und Ceroxid.

3. Öl-in-Wasser-emulgiertes Kosmetikum nach einem der Ansprüche 1 oder 2,
wobei die einer hydrophoben organischen Oberflächenbehandlung unterzogenen anorganischen Pulvermikroteilchen (A) in dem Zustand einer wässrigen Dispersion gemischt werden, die durch Dispergieren derselben in einem wässrigen System unter Verwendung eines nichtionischen oberflächenaktiven Organopolysiloxans erhalten wird.

**Revendications**

1. Cosmétique à émulsification huile dans l'eau contenant des microparticules de poudre inorganique (A) ayant une couche de revêtement primaire contenant de la silice avec une surface la plus externe soumise à un traitement de surface organique hydrophobe et au moins une sorte de substance gonflant à l'eau (B), dans lequel la quantité revêtue de la couche de revêtement primaire est de 1 à 25 % en termes des matières inorganiques totales par rapport à la poudre inorganique totale, et
dans lequel la substance gonflant à l'eau (B) est choisie dans le groupe consistant en l'hectorite, la cellulose produite par microorganisme, la cellulose dérivée de plante, et l'agar-agar.

2. Cosmétique à émulsification huile dans l'eau selon la revendication 1,
dans lequel la microparticule de poudre inorganique est au moins un élément choisi dans le groupe consistant en l'oxyde de titane, l'oxyde de zinc, et l'oxyde de cérium.

3. Cosmétique à émulsification huile dans l'eau selon l'une quelconque des revendications 1 ou 2,
dans lequel les microparticules de poudre inorganique (A) ayant fait l'objet d'un traitement de surface organique hydrophobe sont mélangées dans l'état d'une dispersion aqueuse obtenue en dispersant celles-ci dans un système aqueux à l'aide d'un tensioactif non ionique organopolysiloxane.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013018827 A
- WO 2013018828 A
- JP 2009161496 A
- JP 2011073971 A
- JP H03115211 B
- JP H02247109 B